**Europäisches Patentamt**

**European Patent Office**

**Office européen des brevets**

(11) Veröffentlichungsnummer : **0 115 023**
**B1**

(12) **EUROPÄISCHE PATENTSCHRIFT**

(45) Veröffentlichungstag der Patentschrift :
27.07.88

(51) Int. Cl.⁴ : **C 12 N 9/94**

(21) Anmeldenummer : 83112809.5

(22) Anmeldetag : 20.12.83

(54) Verfahren zur Gewinnung von Pankreatin.

(30) Priorität : 30.12.82 DE 3248587
30.12.82 DE 3248588

(43) Veröffentlichungstag der Anmeldung :
08.08.84 Patentblatt 84/32

(45) Bekanntmachung des Hinweises auf die Patenterteilung : 27.07.88 Patentblatt 88/30

(84) Benannte Vertragsstaaten :
AT BE CH DE FR GB IT LI NL SE

(56) Entgegenhaltungen :
CH-B- 558 814
DE-A- 2 106 706
DE-A- 2 620 289
US-A- 3 223 594

(73) Patentinhaber : **Nordmark Arzneimittel GmbH.**
**Postfach 44**
**D-2082 Uetersen 1 (DE)**

(72) Erfinder : **Schultze, Hans, Dr.**
**Werftweg 21**
**D-2082 Moorrege (DE)**

(74) Vertreter : **Mutzbauer, Helmut, Dr. et al**
**Carl-Bosch-Strasse 38**
**D-6700 Ludwigshafen (DE)**

## Beschreibung

Als Pankreatin bezeichnet man das extrahierte Gemisch der Enzyme der Pankreasdrüse, bestehend i. w. aus Lipasen, Amylase und Proteasen. Pankreatin wird als Wirkstoff zur Behandlung von Verdauungsstörungen, die auf Pankreasinsuffizienz beruhen angewendet. Als Ausgangsmaterial dient hauptsächlich Schweinepankreas in frischem oder gefrorenem Zustand, dem ursprünglich lediglich das Wasser und das Fett entzogen wurde. Wegen der Empfindlichkeit der Enzyme mußte das aber sehr vorsichtig geschehen.

Nach den noch heute üblichen Verfahren wird die Trocknung und Entfettung mit Lösungsmitteln durchgeführt, die Wasser und Fett zugleich lösen, z. B. Aceton oder höheren Alkoholen. Nach anderen bekannten Verfahren werden die beiden Vorgänge nacheinander durchgeführt, meist erst die Trocknung (Gefriertrocknungsverfahren), danach die Entfettung mit einem leicht flüchtigen Fettlösungsmittel ; oder auch umgekehrt : Entfettung in flüssiger Phase mit (Halogen-) Kohlenwasserstoffen und daran anschließend die Trocknung der wäßrigen Phase.

Alle Zubereitungen von Pankreatin, die nach einem dieser Verfahren hergestellt wurden, haben den Nachteil, daß die Proteasen, z. B. Trypsin und Chymotrypsin in ihnen nicht frei vorliegen, sondern in ihrer inaktiven Enzymogenform. Sie können deshalb gerade bei Patienten, die keine körpereigene Enterokinase produzieren, nicht aktiv werden, sind also völlig unwirksam. Ein weiterer Nachteil ist der Fasergehalt dieser Pankreatine, der aus dem Bindegewebe der Drüsen stammt und medizinisch wertlos ist. Dieser Faseranteil stört bei der galenischen Zubereitung nicht nur den Preßvorgang, sondern behindert auch den gewünschten Zerfall des Preßlings am Wirkungsort, so daß die Enzyme zu einem großen Teil ungenutzt bleiben.

Bei einigen neueren Verfahren wird diesen Erkenntnissen bereits Rechnung getragen. Die Fasern werden entfernt und zur Erzeugung aktivierter Enzyme wird eine Autolye durchgeführt. Aber durch die Autolyse werden die Enzyme nicht nur freigesetzt, sondern auch geschädigt. So kommt es, daß bei diesen Verfahren keine volle Enzymausbeute erhalten wird.

Die Durchführung der Autolyse wird sehr unterschiedlich gehandhabt. Bei dem Verfahren der US-PS 3 223 594 wird z. B. 2 Stunden bei Raumtemperatur in Gegenwart von viel Wasser autolysiert, bei den Verfahren der DE-OSen 26 20 289 und 27 16 719 15 Stunden bei 15 °C bzw. 4 Stunden bei 15 °C in Gegenwart von mit Wasser nicht mischbaren Fettlösungsmitteln. Nach der Beschreibung der GB-PS 1 328 202 wird die Autolyse in 1/2 bis 2 Stunden bei 20-30 °C unter Zusatz von Natronlauge oder Ammoniak durchgeführt. In der DE-AS 21 06 706 wird eine Autolyse beschrieben, die, zur Verbesserung der Enterokinaseaktivität, sich bei 4 °C in Gegenwart von Schweineduodenum über 7 Tage erstreckt.

Nach der US-PS 3 223 594 wird eine Ausbeute von 11 Gew.-% mit Enzymeinheiten bis 10 × NF angegeben, was etwa 69 % der möglichen Enzymausbeute entspricht, da im allgemeinen 100 g Drüsengewebe, verlustlos getrocknet und entfettet, 20 g Pankreatin mit einer Qualität von 8 × NF ergeben.

In der GB-PS 1 328 202 findet sich eine Qualitätsangabe von 6 bis 10 × NF, bei einer Gewichtsausbeute von steril gefiltertem Pankreatin von 3,4 %, was also nur 13 bis 21 % der rechnerisch möglichen Enzymausbeute entspricht. In den DE-OSen 26 20 289 und 27 16 719 wird nur bezüglich der Lipase von einer vollen Enzymausbeute berichtet, während die Ausbeuten an Amylase und an Proteasen offenbar so wesentlich niedriger liegen, daß hier gar nicht mehr von Pankreatin gesprochen wird, sondern von einem « lipasereichen Enzympräparat ».

Die rechnerisch mögliche Ausbeute an aktivierten Enzymen ist also bisher offenbar noch nicht erhalten worden. Auch ist noch keine Angabe über das Ausmaß der Proteasenaktivierung gemacht worden, die an sich durch Vergleich der Proteasenaktivität mit und ohne Enterokinaseaktivierung bestimmt werden kann. Ohne Aktivierung werden hier unter 0,5 FIP-E/mg gefunden.

In der vorstehenden Schilderung des Standes der Technik bedeutet NF die Abkürzung für National Formulary, das amtliche amerikanische Arzneibuch, herausgegeben von der American Pharmaceutical Association. Auf Seite 514 der 13. Auflage (NF XIII) wird Pankreatin als eine enzymhaltige Substanz beschrieben, deren Amylase-Aktivität ausreicht, um mindestens das 25-fache Gewicht an Kartoffelstärke in lösliche Kohlenhydrate umzuwandeln und auch das 25-fache Gewicht an Casein zu proteolysieren. Pankreatine mit größerer Verdauungskraft werden mit einem entsprechenden Vielfach-Faktor versehen.

So bedeutet z. B. 4-fach NF (oder 4 × NF) ein Pankreatin, in dem so viel Amylase, Proteasen und Lipase enthalten ist, daß 1 g dieses Pankreatins die Umwandlung von (4 mal seinem 25-fachen Gewicht =) 100 g des jeweils entsprechenden Substrates (Stärke, Casein, Olivenöl) katalysieren kann.

Die Definition der FIP-Einheiten wird von der « Commission on Pharmaceutical Enzymes » unter dem Dachverband der « Fédération Internationale Pharmaceutique » (= FIP) in der Monographie « Pharmaceutical Enzymes » von R. Ruyssen und A. Lauwers, 1978, Scientific Publishing Company, Gent/Belgien, gegeben. Auf S. 22 findet man die allgemeine Aussage, daß 1 FIP-E die Umsetzung von 1 Mikromol bzw. 1 Mikroäquivalent Substrat pro Minute unter sonst wohl definierten äußeren Bedingungen katalysiert oder — bei Fällen in denen das Substrat chemisch nicht hinreichend definiert ist — 1 Mikromol bzw. 1 Mikroäquivalent pro Minute Reaktionsprodukt erzeugt wird. Die zur Bestimmung der einzelnen Enzyme anzuwendenden äußeren Bedingungen werden auf den Seiten 21 bis 84 beschrieben.

Die FIP-Einheiten werden also durch eine Messung der katalysierten Anfangsreaktionsgeschwindigkeit bestimmt und sind deshalb auch schneller (und genauer) zu ermitteln, also die früher gebräuchliche NF-Multiplizität.

Eine brauchbare Umrechnungstabelle von FIP-E auf x-fach NF-Qualität (experimentell) ist nachstehend engegeben, die auch Lipase-Einheiten erfaßt, obwohl eine Lipase-Bestimmung im NF XIII nicht beschrieben ist. Dabei ist angenommen, daß ein Pankreatin-Präparat, das bezüglich der Amylase z. B. als 6-fach NF analysiert wurde, auch bezüglich Proteasen und Lipase 6-fach NF sei, das Aktivitätsverhältnis der Enzyme im natürlichen Schweinepankreas und dem daraus hergestellten Pankreatin-Präparat also als konstant angesehen wird.

Der praktische Nutzen bei der Angabe des NF-Faktors ist die durch eine einzige Zahl ausdrückbare Pankreatin-Qualität hinsichtlich der Aktivitäten aller darin enthaltenen Enzyme.

Es entspricht erfahrungsgemäß :

1-fach NF = 6 250 FIP-E/g Pankreatin an Amylase
= 5 000 FIP-E/g Pankreatin an Lipase
=   375 FIP-E/g Pankreatin an Trypsin
= 1 250 FIP-E/g Pankreatin an Chimotrypsin

Durch Verdopplung der FIP-Werte gelangt man zu 2-fach NF usw.

Bei den bekanntgewordenen Verfahren, die von einem Autolysat ausgehen, fällt das Pankreatin nie in einer Form an, die sich problemlos weiterverarbeiten läßt. Auch Verfahren, nach denen aus Pankreas unmittelbar Pankreatin, und zwar von hohem Schüttgewicht gewonnen wird, existieren in strengem Sinne nicht ; man könnte allenfalls die Herstellung eines « lipasereichen Enzympräparats » nach den vorstehenden DE-OSen hierzu rechnen, die allerdings mit erheblichem technischen Aufwand und 3 verschiedenen Lösungsmitteln arbeitet.

Aufgabe der Erfindung ist es daher, ein möglichst einfaches und ungefährliches Verfahren anzugeben, mit dem man ohne großen technischen Aufwand und bei voller Enzymausbeute Pankreatin von höchster Enzymaktivität und mit freien Proteasen erhält, das darüber hinaus keimarm, rieselfähig, von hohem Schüttgewicht und faserfrei ist und das sich praktisch ohne Verluste an Enzymen verarbeiten und lagern läßt, wobei die wesentlichen Schritte das Autolysieren eines wäßrigen, gegebenenfalls calciumionenhaltigen Pankreasgewebebreies bzw. einer wäßrigen Gewebesuspension, Entfernen der Faseranteile sowie gegebenenfalls Entwässern, Entfetten, Ausfällen und Trocknen der erhaltenen Fällung umfassen.

Diese Aufgabe wird erfindungsgemäß gelöst, indem man die Autolyse unter Zusatz von bis zu 20 Gew.-% Isopropylalkohol, bezogen auf die Suspension, bei einem pH-Wert von 6,5 bis 8,5, vorzugsweise in Gegenwart eines pH 6,5 bis 7,5 einstellenden Puffers, z. B. bis 3 % und insbesondere 1 bis 1,5 % Alkalihydrogencarbonat, bezogen auf eingesetztes Gewebe und ggf. in Gegenwart einer geringen Menge eines Calcium-Salzes unter 30 °C durchführt und sie unterbricht, sobald eine Probe der Autolysensuspension in 55 bis 65 %igem wäßrigem Isopropylalkonol eine Sedimentationsgeschwindigkeit von etwa 3 bis 10 mm in 1 bis 3 Minuten erreicht. Diese Angabe bezieht sich auf die freie Sedimentation im Erdschwerefeld, also ohne Zentrifugieren.

Die Beendigung der Autolyse tritt ein durch Zugabe von Isopropylalkohol zur Hauptmenge an Autolysat bis zu einer Konzentration von 30 bis 35 Gew.-% — bezogen auf die Summe von Wasser und Isopropylalkohol ; das hierdurch gleichzeitig dünnflüssig gewordene Autolysat kann von den Bindegewebsfasern abgesiebt werden. Man trägt es bei 15 bis 25 °C in vorgelegten Isopropylalkohol ein, so daß eine 55 bis 65 %ige Lösung — wiederum bezogen auf die Summe Wasser und Isopropylalkohol — entsteht, aus der das Pankreatin in grober, körniger Form ausfällt und sofort sedimentiert. Nach Abhebern des Überstandes kann man das sedimentierte Pankreatin durch mehrfaches Aufrühren mit Isopropylalkohol und Absitzenlassen fett- und tryptonfrei waschen. Der Niederschlag soll schließlich eine Endkonzentration zwischen 70 bis 85 — vorzugsweise etwa 75 bis 76 Gew.-% — an Isopropylalkohol aufweisen und kann nach Absaugen oder Abschleudern vorzugsweise unter vermindertem Druck und Behandlung mit trockener Luft (z. B. Gehalt von unter 20 % relativer Feuchte bei 20 °C) oder mit Stickstoff getrocknet werden. Das vorstehend beschriebene Waschen des Niederschlages kann auch mit Aceton anstelle von Isopropylalkohol geschehen.

Bei den vorstehenden Angaben zum Alkoholgehalt der wäßrigen Lösungen bzw. Überstände wird, soweit das Gewicht des eingesetzten Rohmaterials in die Berechnung eingeht, dessen Wassergehalt mit 63 Gew.-% angenommen.

Vor allem für die zuletzt angegebene Endkonzentration an Isopropanol im Niederschlag muß beachtet werden, daß die Gleichgewichtseinstellung zwischen Niederschlag und Überstand sehr lange dauern kann, da Eiweißkörper Wasser sehr zäh festhalten und daher innerhalb der gefällten Partikel zunächst eine wasserreichere Phase sich hält. Auch diese Endkonzentrations-Angaben beziehen sich daher nicht auf die analytische Zusammensetzung der Lösungsphase, sondern sind wie vorstehend angegeben, berechnet.

Praktisch verfährt man bei der Durchführung des erfindungsgemäßen Verfahrens wie folgt : Das gewolfte oder gecutterte Pankreas wird zur besseren Rührfähigkeit mit 20 bis 80 % Wasser versetzt. Der Autolysevorgang wird durch etwa 1 bis 1,5 % Natriumbicarbonat schonend, d. h. ohne daß eine Schädigung der Enzyme stattfindet, in erwünschter Weise gelenkt und beschleunigt ; ein Zusatz von mehr als 3 % Bicarbonat könnte die Lagerbeständigkeit des Pankreatins gefährden. Der Zusatz einer geringen Menge eines Calciumsalzes (z. B. unter 1 %) begünstigt die Autolyse. Mit der Freisetzung der Proteasen aus ihren Proenzymen beschleunigt sich durch die ständig anwachsende Menge freier Proteasen zunächst die Verdauung der begleitenden Pankreas-Eiweißstoffe.

Die Verdauung würde nun aber schließlich auf die empfindlichen Enzyme übergreifen, so daß besonders die Amylase und auch die Proteasen selbst geschädigt würden. Speziell zur Schonung der Proteasen werden bis zu 20 % Isopropylalkohol zum Gemisch zugefügt, vorzugsweise etwa 10 bis 15 %. Dieser Zusatz alleine reicht jedoch nicht aus, um die Enzymaktivitäten voll zu erhalten. Es ist daher wichtig, den Zeitpunkt kurz vor der beginnenden Enzymschädigung herauszufinden, zu dem man die Autolyse am günstigsten unterbricht. Die Autolysedauer bis zu diesem Zeitpunkt schwankt nämlich auch unter sonst völlig gleichen Bedingungen um bis zu 600 % je nach Herkunft, Alter und Lagerung der Pankreasdrüsen, so daß eine allgemeine Zeitangabe nicht möglich ist.

Erfindungsgemäß läßt sich der günstigste Zeitpunkt dadurch festzustellen, daß man mit einem einfachen Schnelltest an einer Probe des Autolysats die optimale Sedimentationsgeschwindigkeit (Absitzgeschwindigkeit) in 55 bis 65 %igem Isopropylalkohol ermittelt. Dieser Absitztest ist praktisch die an kleinen Proben vorvollzogene weitere Verarbeitung des Autolysats bis zur Pankreatinfällung und Beobachtung über deren sich ständig veränderndes Sedimentationsverhalten. Hierzu kann man z. B. in Abständen von 1/2 Stunde — gegen Schluß auch z. B. alle 10 oder 15 Minuten — jeweils 10 g der Autolysatsuspension entnehmen, mit 5 ml 84 %igem Isopropylalkohol verrühren und die entstandene Lösung unter Rühren in 20 ml 84 %igen Isopropylalkohol eingießen. Man bestimmt die Sedimentationsgeschwindigkeit des ausgefällten Niederschlages. Wenn nach 3 Minuten Absitzzeit im 50 ml-Becherglas z. B. mehr als 3 bis 5 mm klare Oberschicht erkennbar wird, ist in der Regel die optimale Sedimentationsgeschwindigkeit erreicht und damit auch der gesuchte Zeitpunkt vor der Enzymschädigung. Die beginnende Enzymschädigung kann daran erkannt werden, daß der Überstand (die Oberschicht) nicht mehr klar ist (bzw. das Sediment vom Überstand durch eine getrübte zone getrennt ist).

Zur Erzielung eines einheitlicheren Autolyseverhaltens unterschiedlicher Chargen ist es günstig, etwas Pankreatin aus einem früheren Ansatz zuzufügen.

Die Autolyse kann bei Temperaturen zwischen — 2° bis + 30°, vorzugsweise unter 25 °C durchgeführt werden, wobei die Einhaltung einer konstanten Temperatur nicht erforderlich ist. Hierdurch ergibt sich eine Fülle von Nutzungsmöglichkeiten, wie Energie-Einsparung, Nacht- und Wochenend-Autolyse, Arbeitszeiteinsparung. Der günstigste Endpunkt wird immer an dem Absetztest erkannt.

Der gesamte Ansatz wird nun mit weiterem Isopropylalkohol versetzt, um die Autolyse zu unterbrechen. Nach dem erfindungsgemäßen Verfahren wird durch kurzes Rühren der Mischung bei einem Gehalt von 30 bis 35 % an Isopropylalkohol (bezogen auf Wasser und Alkohol) eine fast klare und so dünnflüssige Lösung neben ungelösten Fasern erhalten, daß die Abtrennung der Fasern, auch bei großen Ansätzen völlig problemlos durch einen Siebkorb von 4 bis 5 mm Maschenweite gelingt, der am besten mit einem Rührer versehen wird, um zuletzt nur noch trockenen Faserrückstand übrig zu behalten. Die abgesiebte Lösung läßt man zur Pankreatinausfällung in vorgelegten Isopropylalkohol einlaufen, der zwischen 80 und 100 % Gehalt haben kann, aber in einer Menge vorliegt, die ausreicht, um nach Vermischen mit der 30 bis 35 %igen Lösung eine Fällungskonzentration von 55 bis 65 % Isopropylalkohol zu erreichen. Aus wäßriger Lösung rückgewonnener, sog. 84 %iger Isopropylalkohol, der — angenähert dem Azeotrop von 88 Gew.-% — etwa 84 % Alkoholgehalt hat, läßt sich mit Vorteil verwenden.

Bei Konzentrationen von unter 55 % ist die Fällung unvollständig, würde also einen Ausbeuteverlust mit sich bringen. Bei über 65 % werden vermutlich unerwünschte Enzyminhibitoren mit ausgefällt, wodurch die Aktivität der Proteasen niedriger erscheint.

Die nach dem Verfahren der Erfindung erhaltene Pankreatinfällung ist so grobkörnig, daß sie sich innerhalb einer Stunde auf 10 bis 20 % des ursprünglichen Volumens abgesetzt hat, und man die klare, überstehende Lösung einfach abhebern oder abpumpen kann. Der abgesetzte Niederschlag kann durch Aufrühren mit zurückgewonnemen 84 %igem, zuletzt mit frischem absolutem Isopropylalkohol und jeweils erneutem Sedimentieren auf einfache Weise gewaschen werden. Diese Absetzmethodik ist zwar schon in der GB-PS 1 328 202 beschrieben, konnte aber keine Bedeutung erlangen, weil das Sedimentieren meist nicht gelang. Vielmehr ist erst durch den erfindungsgemäßen Absetztest ein sicheres Verfahren möglich.

An dieser Stelle würde nach anderen üblichen Verfahren eine geschlossene Zentrifuge mit Inertgas-Schutz gebraucht, die das Produkt durch Erwärmung schädigt, eine wesentlich längere Zeit für die Abtrennung benötigt und ein Waschen des Pankreatins wäre nur nach intensivem Zerkleinern in frischem Lösungsmittel (mit der Gefahr örtlicher Überhitzungen) und nachfolgendes erneutes Zentrifugieren möglich, wobei viel Energie und Arbeitszeit gebraucht wird.

Aus den abgeheberten Überständen kann der Isopropylalkohol durch Destillation zurückgewonnen werden. Der Destillationsrückstand trennt sich in eine untere, wäßrige Schicht, die Tryptone und Seife gelöst enthält, und eine darauf schwimmende Fettschicht, die nach Erkalten erstarrt. Der rückgewonnene 84 %ige Isopropylalkohol kann an jeder Stelle des erfindungsgemäßen Verfahrens direkt wieder eingesetzt werden.

Die Einstellung eines Isopropylalkoholgehalts von 70 bis 85 % ist für den im folgenden beschriebenen Trocknungsprozeß günstig ; anstelle von Isopropylalkohol könnte auch Aceton in einer Konzentration von 80 bis 95 % dienen, wobei aber das Lösungsmittel gewechselt werden müßte.

Der erhaltene Pankreatin-Absetzrückstand läßt sich hervorragend filtern und kann daher am einfachsten in einer Nutsche abgesaugt werden. Natürlich läßt sich auch ein selbsttätig arbeitendes Filter oder eine automatische Zentrifuge verwenden. Man kann den Rückstand in herkömmlicher Weise z. B. durch Gefriertrocknung haltbar machen ; vorzugsweise wird aber wie folgt verfahren :

Während der Trocknung des Kuchens, welcher anhaftende Mutterlauge mit einem Gehalt von etwa 70 bis 85 % — vorzugsweise 75 bis 80 % — Isopropylalkohol enthält, wird eine starke Volumenkontraktion beobachtet. Dieser Schrumpfungsprozeß führt zu einem Pankreatin von hohem Schüttgewicht (0,5 bis 0,7 g/ml), das nach Grobvermahlung gut rieselfähige Körnchen bildet. Enthält dagegen das zu trocknende Pankreatin Isopropylalkohol von deutlich über 85 bis 86 % Gehalt, so erfolgt keine Kontraktion und es entsteht ein Produkt mit so niedrigem Schüttgewicht (0,2 g/ml), daß es sich leicht elektrostatisch auflädt und somit schlecht zu handhaben ist. Enthält andererseits das zu trocknende Pankreatin Isopropylalkohol von unter 70 %, so erfolgt zwar die gewünschte Volumenkontraktion, aber die Lipase erleidet eine gewisse Schädigung, selbst wenn die Trocknung bei 0 °C unter vermindertem Druck durchgeführt wird. Eine Enzymschädigung kann auch eintreten, wenn bei über 25 °C getrocknet wird, oder wenn — mit oder ohne Anwendung von vermindertem Druck — die Trocknung mit Luft vorgenommen wird, die bei 20 °C mehr als 20 % relative Feuchte enthält. Die vorstehenden Angaben sind etwas vom tatsächlichen Gehalt an Isopropylalkohol abhängig.

Das erfindungsgemäß hergestellte Pankreatin ist keimarm. Die Keime verbleiben größtenteils in den Fasern, von denen die Pankreatinlösung abgesiebt wird. Außerdem ist die Einwirkung von Isopropylalkohol in der benötigten Konzentration bekanntlich eine Maßnahme zur Abtötung von Keimen (siehe die CH-PS 558 814), insbesondere, weil die in der anfangs rein wäßrigen Suspension gegebenenfalls vorhandenen Sporen während der Autolyse in ihre empfindliche, vegetative Form übergehen.

Die gute Beständigkeit des erfindungsgemäß hergestellten Pankreatins wird in einem Belastungstest deutlich, der einer weiteren Verarbeitung zu Dragees Rechnung trägt : Die Belastung besteht darin, daß das Pankreatin zunächst an offener Luft von 20 °C und 58 % relativer Feuchte 20 Stunden gelagert wird und danach im geschlossenen Gefäß 24 Stunden auf 50 °C erwärmt wird.

Tabelle 1

Vergleich der Amylase- und Lipase-Stabilität von Pankreatinen, die nach dem Stand der Technik und nach dem erfindungsgemäßen Verfahren hergestellt wurden. Die Pankreatine 1, 2 und 3 sind handelsübliche Präparate von verschiedenen Herstellern

| Herstellverfahren | Amylase Ausgangs- aktivität in FIP-E/ mg | Belastungs- verlust | Lipase Ausgangs- aktivität in FIP-E/ mg | Belastungs- verlust |
|---|---|---|---|---|
| 1. Stand d. Technik | 43,1 | 6 % | 40,7 | 7 % |
| 2. Stand d. Technik | 57,2 | 43 % | 37,0 | 14 % |
| 3. Stand d. Technik | 51,0 | 41 % | 48,3 | 31 % |
| 4. erfindungsgemäßes Verfahren | 93,6 | 3 % | 90,3 | 6 % |

Die Tabelle 1 zeigt, daß bei herkömmlichen Pankreatinen die Verluste bei Belastung stark mit der Enzymaktivität ansteigen. Überraschenderweise ergibt jedoch das nach dem erfindungsgemäßen Verfahren hergestellte Pankreatin trotz wesentlich höherer Enzymaktivität den geringsten Verlust bei Belastung.

Beispiel

100 kg tiefgefrorenes Schweinepankreas werden gewolft oder gecuttert und im 250 l-Kessel mit einer Lösung von 100 g Calciumgluconat in 20 l Wasser zusammen mit 200 g Silicone-Entschäumer verrührt. Je nach dem Gehalt der Drüse an freiem Trypsin werden bis zu 1 kg fertiges Pankreatin, gelöst in 5 l Wasser, als Starthilfe für die Autolyse zugegeben. Diese Pankreatinmenge wird durch die später beschriebene « 37°-Hydrolysenprobe » ermittelt. Zu diesem Zweck werden an dieser Stelle 120 g aus dem Ansatz entnommen.

5

Nach Zugabe von 15 l 84%igem Isopropylalkohol wird weiterhin eine Lösung von 1,5 kg Natriumhydrogencarbonat in 20 l Wasser eingerührt. Man läßt über Nacht stehen, wobei die Temperatur etwa von 2° auf 12° ansteigt, erwärmt am nächsten Morgen auf 20° und rührt bei dieser Temperatur weiter.

Den Endpunkt der Autolyse erkennt man an dem Absitztest. Hierzu werden während dieses letzten Abschnitts der Autolyse zunächst alle halbe, später alle Viertelstunde 10 g der Suspension entnommen, mit 5,4 ml 84%igem Isopropylalkohol mittels eines Glasstabes eine Minute verrührt, die entstehende Lösung von den am Glasstab klebenden Fasern abgetrennt und in 20 ml einer im 50 ml-Becherglas mit Magnetrührer vorgelegten Lösung von 84%igem wäßrigem Isopropylalkohol eingerührt. Nach einer Minute stellt man den Rührer ab und läßt eine bzw. drei Minuten lang. absitzen.

Die Probe wird positiv bewertet und daher die Autolyse beendet, sobald in einer Minute 3 mn bzw. in drei Minuten 10 mm (mehr oder weniger klare) Oberschicht erkennbar sind.

Als Beispiel wird eine typische Absitztestreihe wiedergegeben, die einsetzt, sobald das Autolysat 20 °C aufweist.

Absitztest

| Autolysendauer-bei 20°C | mm/0,5 Min. | entspricht mm/1 Min. | entspricht mm/3 Min. |
|---|---|---|---|
| 1,00 Std. | 0 | 0 | 0 |
| 2,00 Std. | 0 | 0 | 0 |
| 2,50 Std. | 0 | 0 | 1 |
| 2,75 Std. | 0 | 1 | 4 |
| 3,00 Std. | 1 | 3 | 10 |
| 3,25 Std. | 3 | 6 | 18 |
| 3,50 Std. | 5 | 10 | 20 |
| 3,75 Std. | 9 | 18 | 20 |
| 4,00 Std. | 14 | 20 | 20 |

In diesem Beispiel wird also der günstigste Zeitpunkt zur Beendigung der Autolyse nach 3 Stunden erreicht. Damit dieses auch erlaubt — wenigstens ungefähr bei Verwendung einer anderen, unbekannten (z. B. länger oder kürzer gelagerten) Schweinepankreaspräparats vergleichbare Verhältnisse zu erzielen, wird die « 37°-Hydrolysenprobe » durchgeführt : Aus dem mit Ca-gluconat versetzten Drüsenbrei werden 120 g entnommen, mit 1,5 g Natriumhydrogencarbonat in 25 ml Wasser versetzt und 30 Minuten bei 37 °C gerührt. Mit einer Probe dieses Schnellautolysats wird der Absitztest durchgeführt. Bei unter 2 mm/3 Min. werden 2 Mio E freies Trypsin, bei bis zu 12 mm/1 Min. wird 1 Mio E und bei noch höherer Absatzgeschwindigkeit kein freies Trypsin zu dem Hauptansatz (in Form von Pankreatin) zugesetzt. (1 Mio E freies Trypsin sind z. B. in 250 g Pankreatin mit 4 000 E/g enthalten). Auf diese Weise kann man Rohmaterialien unterschiedlicher Herkunft für die Betriebspraxis standardisieren.

Nachdem nun der günstigste Zeitpunkt ermittelt ist, wird die Autolyse des Ansatzes durch Einpumpen von 72,5 l 84%igem (rückgewonnemen) Isopropylalkohol beendet. Man rührt noch eine halbe Stunde, wobei ein klare Lösung neben ungelösten Fasern entsteht, von denen dann abgesiebt wird. Hierzu wird der Kesselinhalt in einen offenen 50 l Kessel mit eingelegtem Sieb von ca. 5 mm Maschenweite abgelassen, der mit einem Ankerrührer versehen ist. Der Bodenauslauf des Siebkessels ist über eine Pumpe mit einem 500 l-Polypropylenbehälter verbunden. Er ist mit 318 l rückgewonnenem 84%igem Isopropylalkohol beschickt und wird nun unter langsamem Rühren mit der abgelassenen, gesiebten Lösung weitergefüllt. Die im Siebkessel zurückbleibenden Fasern werden noch 10 Min. lang trocken gerührt. Das Gewicht der Fasern beträgt 7 bis 8 kg.

In dem 500 l-Behälter setzt sich das ausgefällte Pankreatin als grober Niederschlag in einer Stunde auf ein Volumen von 80 l ab (bei 20 °C ; bei 24 °C in einer halben Stunde). Der Überstand wird abgehebert und der Rückdestillation zugeführt. Den Bodensatz verrührt man mit 63 l rückgewonnemen 84%igem Isopropylalkohol und läßt über Nacht nochmals absitzen. Am nächsten Tag wird dieser Vorgang wiederholt und der so entstandene Bodensatz mit etwa 45 l 84%igem Isopropylalkohol versetzt, d. h. soviel, wie nötig, um eine Pankreatinsuspension in 76%igem Isopropylalkohol herzustellen. Diese Suspension wird über eine Nutsche von 40 cm Durchmesser gefiltert und gut trocken gesaugt.

Der Filterkuchen wird im Cutter kurz zerkleinert und auf Bleche ausgebreitet. Die Trocknung erfolgt über Nacht bei Heiztemperaturen bis 55 °C unter einem Vakuum von etwa 5 mbar.

Pankreatin-Ausbeute : 11,6 kg
Amylase-Aktivität : 93,6 FIP-E/mg

| | |
|---|---|
| Lipase-Aktivität : | 90,3 FIP-E/mg |
| Protease-Aktivität mit Enterokinase aktiviert : | 5,6 FIP-E/mg |
| ohne Aktivierung : | 5,6 FIP-E/mg |
| Trypsin-Aktivität mit Enterokinase aktiviert : | 4,2 FIP-E/mg |
| ohne Aktivierung : | 4,1 FIP-E/mg |
| Chymotrypsin-Aktivität mit Enterokinase aktiviert : | 28,3 FIP-E/mg |
| ohne Aktivierung : | 28,3 FIP-E/mg |
| Trockengehalt : | 98,7 % |
| Fettgehalt : | 0,4 % |
| Schüttdichte : | 0,65 g/ml |
| Stampfdichte : | 0,76 g/ml |
| Keimzahl : | 200 Keime/g |
| | keine nach USP XVII |
| | unerwünschten Keime |

## Patentansprüche

1. Verfahren zur Gewinnung von Pankreatin durch Autolysieren einer wäßrigen, gegebenenfalls Calciumionen enthältenden Pankreasgewebesuspension. Entfernen der Faseranteile, gegebenenfalls Entwässern, Entfetten, Ausfällen und Trocknen der erhaltenen Fällung, dadurch gekennzeichnet, daß man die Autolyse unter Zusatz von bis zu 20 Gew.-% Isopropylalkohol, bezogen auf die Suspension, bei einem pH-Wert von 6,5 bis 8,5 unter 30 °C vornimmt und unterbricht, sobald eine Probe in 55 bis 65 %igem wäßrigem Isopropylalkohol eine im Erdschwerefeld gemessene Sedimentationsgeschwindigkeit von etwa 3 bis 10 mm/l bis 3 min erreicht.

2. Verfahren nach Anspruch 1, dadurch gekennzeichnet, daß man die Einstellung des pH-Wertes mit bis zu 3 %, bezogen auf eingesetztes Gewebe, an Alkalihydrogencarbonat vornimmt.

3. Verfahren nach Anspruch 1, dadurch gekennzeichnet, daß man die Autolyse in Gegenwart von Pankreatin aus einem früheren Ansatz vornimmt.

4. Verfahren nach Anspruch 1, dadurch gekennzeichnet, daß man die Autolyse durch Zugabe von Isopropylalkohol bis zu einer Konzentration von 30 bis 35 % — bezogen auf die Summe von Wasser und Isopropylalkohol — unterbricht, die dünnflüssig gewordene Zubereitung von den Bindegewebsfasern absiebt, die Lösung bei 15 bis 25 °C in soviel Isopropylalkohol einträgt, daß eine an Isopropylalkohol 55 bis 65 gew.%ige wäßrige Lösung entsteht, das ausgefallene Pankreatin in körniger Form sedimentieren läßt, nach Abheben des Überstandes durch gegebenenfalls mehrfaches Aufrühren mit Isopropylalkohol und Sedimentieren fett- und tryptonfrei wäscht und vorzugsweise unter vermindertem Druck trocknet.

5. Verfahren nach Anspruch 4, dadurch gekennzeichnet, daß man das ausgefällte Pankreatin sedimentieren läßt und nach Abhebern oder Absaugen des Überstandes durch gegebenenfalls mehrfaches Aufrühren mit Isopropylalkohol oder Aceton fett- und tryptonfrei wäscht, bis ein Niederschlag mit einer Konzentration zwischen 70 und 85 % an Isopropylalkohol bzw. 80 bis 95 % an Aceton entstanden ist, den man absaugt oder abschleudert und unter Behandlung mit trockener Luft oder Stickstoff bei unter 25 °C vorzugsweise unter vermindertem Druck trocknet.

## Claims

1. A process for isolating pancreatin by autolysis of an aqueous pancreas tissue suspension which may or may not contain calcium ions, removing the fiber constituents and, if desired, dehydrating, defatting, precipitation and drying of the precipitate obtained, wherein the autolysis is carried out at below 30 °C with addition of up to 20 % by weight of isopropyl alcohol, based on the suspension, at a pH of from 6.5 to 8.5, and is stopped as soon as a sample in 55-65 % strength aqueous isopropyl alcohol shows a sedimentation rate, measured under gravity, of about 3-10 mm/1-3 minutes.

2. A process as claimed in claim 1, wherein the pH is adjusted by adding up to 3 %, based on tissue employed, of an alkali metal bicarbonate.

3. A process as claimed in claim 1, wherein the autolysis is carried out in the presence of pancreatin from an earlier batch.

4. A process as claimed in claim 1, wherein the autolysis is stopped by adding isopropyl alcohol up to a concentration of as high as 30-35 %, based on the sum of water and isopropyl alcohol, the batch which has thus acquired a low viscosity is sieved to remove connective tissue fibers, the solution is introduced, at 15-25 °C, into an amount of isopropyl alcohol such that an aqueous solution containing 55-65 % by weight of isopropyl alcohol results, the precipitated pancreatin is allowed to sediment in granular form, the supernatant liquor is siphoned off and the pancreatin is washed free from fat and tryptone, if appropriate, by repeatedly stirring it with isopropyl alcohol and allowing it to sediment, and is dried, preferably under reduced pressure.

5. A process as claimed in claim 4, wherein the pancreatin which has precipitated is allowed to sediment, the supernatant liquor is siphoned off or suction-filtered off, the pancreatin is washed free from fat and tryptone by, if appropriate, repeatedly stirring it with isopropyl alcohol or acetone until a precipitate having an isopropyl concentration of 70-85 % or an acetone concentration of 80-95 % results, and this precipitate is isolated by suction filtration or centrifuging and is dried by treatment with dry air or nitrogen at below 25 °C, preferably under reduced pressure.

## Revendications

1. Procédé d'obtention de pancréatine par autolyse d'une suspension aqueuse de tissu pancréatique, contenant éventuellement des ions calcium, élimination des parties fibreuses, éventuellement déshydratation, dégraissage, précipitation et séchage du précipité obtenu, caractérisé par le fait que l'on effectue l'autolyse à 30 °C, à un pH de 6,5 à 8,5, avec addition de jusqu'à 20 % en poids d'alcool isopropylique, pourcentage rapporté à la suspension, et qu'on l'interrompt dès qu'un échantillon dans de l'alcool isopropylique aqueux, à 55-65 %, atteint une vitesse de sédimentation, mesurée dans le champ de l'attraction terrestre, d'environ 3 à 10 mm/l à 3 min.

2. Procédé selon la revendication 1, caractérisé par le fait que l'on effectue le réglage du pH avec jusqu'à 3 %, rapportés au tissu utilisé, d'hydrogénocarbonate alcalin.

3. Procédé selon la revendication 1, caractérisé par le fait que l'on effectue l'autolyse en présence de pancréatine provenant d'un dépôt antérieur.

4. Procédé selon la revendication 1, caractérisé par le fait que l'on interrompt l'autolyse par addition d'alcool isopropylique jusqu'à une concentration de 30 à 35 %, rapportée à la somme de l'eau et de l'alcool isopropylique, on sépare par tamisage, de la préparation devenue fluide, les fibres de tissu conjonctif, on introduit la solution, à 15 à 25 °C, dans suffisamment d'alcool isopropylique pour qu'apparaisse une solution aqueuse à 55 à 65 % en poids d'alcool isopropylique, on laisse sédimenter sous forme granuleuse la pancréatine précipitée, après enlèvement de la couche supérieure on élimine par lavage la graisse et la tryptone en agitant, éventuellement plusieurs fois, avec de l'alcool isopropylique et en faisant sédimenter, puis on sèche avantageusement sous pression réduite.

5. Procédé selon la revendication 4, caractérisée par le fait que l'on fait sédimenter la pancréatine précipitée et, après enlèvement ou essorage de la couche supérieure, on élimine par lavage la graisse et la tryptone en agitant, éventuellement plusieurs fois, avec de l'alcool isopropylique ou de l'acétone, jusqu'à obtention d'un dépôt d'une concentration de 70 à 85 % en alcool isopropylique ou 80 à 95 % en acétone, dépôt que l'on essore et centrifuge et qu'on sèche en traitant avec de l'air ou de l'azote secs, à une température inférieure à 25 °C, de préférence sous pression réduite.